# EUROPEAN PATENT APPLICATION

(11) **EP 3 610 776 A1**
(43) Date of publication of application: **19.02.2020**
(21) Application number: 18188946.0
(22) Date of filing: 14.08.2018
(51) Int. Cl.: A61B 1/005

(54) **ARTICULATED SEGMENTED INSTRUMENT**

(71) Applicant: ETH Zurich, 8092 Zurich (CH); Universität Zürich, 8006 Zürich (CH)
(72) Inventor: NELSON, Bradley, 8126 Zumikon (CH); CHAUTEMS, Christophe, 8046 Zürich (CH); BIRO, Peter, 8706 Meilen (CH); BOEHLER, Quentin, 8057 Zürich (CH); GAGE, David, 8903 Birmensdorf (CH)

(57) **Abstract**

An articulated segmented instrument comprises an elongate body in the longitudinal direction with a plurality of interconnected links (11). Each link (11) has a central working channel (30) and comprises at least three angulation wire thru holes (22) to accommodate angulation wires to direct a distal tip segment of the instrument in a predetermined direction. Each link (11) comprises two radially opposite protrusions (15) and two opposite receptions (25) in the cylindrical sleeve opposite to the direction of the protrusions (15) and having pivot concave contact surfaces (28) complementary to convex pivot contact surfaces (18). The reception adjacent surface (26) of the cylindrical sleeve creates an articulating angle with the protrusion adjacent surface (16) of the cylindrical sleeve of the adjacent interconnected link (11) through inclination of at least one of the reception adjacent surface (26) or the protrusion adjacent surface (16) of the adjacent interconnected link (11) in the longitudinal direction towards the body of the interconnected link (11) or the adjacent interconnected link (11), respectively.

## Description

### TECHNICAL FIELD

The present invention relates to an articulated segmented instrument comprising an elongate body having a plurality of interconnected links.

### PRIOR ART

US 2011/0295065 A1 discloses a number of different segmented instrument comprising an elongate body having a plurality of links, wherein these links are interconnected via hinges connecting a pair of adjacent links in the plurality of links. These links may have a brake assembly, a lockable and articulable joint. This joint is adapted and configured to increase the number of frictional surfaces available between the pair of adjacent links.

US 2003/0083550 A1 shows a bent tube with joint rings spacedly arranged, side by side, in a row and a connection mechanism which is bridgingly disposed between one of the adjacent joint rings and the other. The connecting mechanism has a pair of hinge members which are rotatably connected around a rivet. The hinge is fixed to a groove as hinge attaching portion formed in one of the adjacent joint rings by soldering, and the hinge member is fixed to a groove formed in the other joint ring by soldering.

The prior art segmented instruments lack structural stability particularly at smaller scales.

### SUMMARY OF THE INVENTION

Based on this prior art it is an object of the present invention to provide a segmented instrument, especially a catheter or an endoscope, that require or can be improved by a steerable control with one or two degrees of freedom. This is especially in need for small scale instruments having a diameter of 1cm or less. This object is achieved with an instrument having the features of claim 1.

An articulated segmented instrument comprises an elongate body in the longitudinal direction of the segmented instrument between a proximal base segment and a distal tip segment having a plurality of interconnected links. The proximal base segment and the distal tip segment each have one interface according to the invention to be articulated with the intervening interconnected links provided between the proximal base segment and the distal tip segment. The interconnected links are hollow cylindrical sleeves with a central working channel and comprise at least three angulation wire thru holes to accommodate each one an angulation wire to direct the distal tip segment in a predetermined direction. For that each interconnected link comprises two radially opposite protrusions in the cylindrical sleeve extending in the longitudinal direction and having a common joint axis defining a convex pivot contact surface over an angle area of more than 180 degrees around the common joint axis providing a neck portion in the longitudinal direction. Each interconnected link also comprises two opposite receptions in the cylindrical sleeve extending in the longitudinal direction opposite to the direction of the protrusions and having a common joint axis defining a concave pivot contact surface over an angle area of more than 180 degrees around the common joint axis providing a passage portion, wherein the pivot concave contact surface is complementary to the convex pivot contact surface and wherein the neck portion is complementary to the passage portion. In other words the protrusions allow two adjacent links to be pivoted around this common joint axis. The convex pivot contact surface over an angle area of more than 180 degrees ensures that there is a neck portion and that the two adjacent links are in a positive fit with just enough play to pivot.

Finally the reception adjacent surface of the cylindrical sleeve creates an articulating angle with the protrusion adjacent surface of the cylindrical sleeve of the adjacent interconnected link through inclination of at least one of the reception adjacent surface or the protrusion adjacent surface of the adjacent interconnected link in the longitudinal direction towards the body of the interconnected link or the adjacent interconnected link, respectively. On the other side, the protrusion adjacent surface of the cylindrical sleeve creates an articulating angle with the reception adjacent surface of the cylindrical sleeve of the adjacent interconnected link through inclination of at least one of the protrusion adjacent surface or the reception adjacent surface of the adjacent interconnected link in the longitudinal direction towards the body of the interconnected link or the adjacent interconnected link, respectively. In other words, the two opposite protrusion and reception adjacent surfaces have a relationship generating this articulating angle, defining the maximum pivoting angle for two adjacent links into one pivoting direction. The pivoting deflection is not necessarily symmetric about the neutral state, i.e. straightly aligned segments of the instrument, when the two opposite surfaces, on either segment, are inclined in a different angle.

The common joint axis of the protrusions can have an angle of 90 degrees with the common joint axis of the receptions, so that for three links in a row one connection can be pivoted in one direction whereas the following connection can be pivoted in a direction perpendicular to the first pivotal direction, so that any point can be reached by a distal tip.

When the common joint axis of the protrusions has an angle of 60 and 120 degrees with the common joint axis of the receptions, a different sequence of pivotal movements can be created.

If the angulation thru holes are rotated by a few degrees for every segment, then a helix-like sequence of the links can also be created.

Preferably, there are four angulation wire thru holes to accommodate each one an angulation wire and wherein the angulation wire thru holes are provided in an angle of 90 degree in the longitudinal direction so that subsequent links can be easily mounted and comprise a symmetric disposition of guide wires.

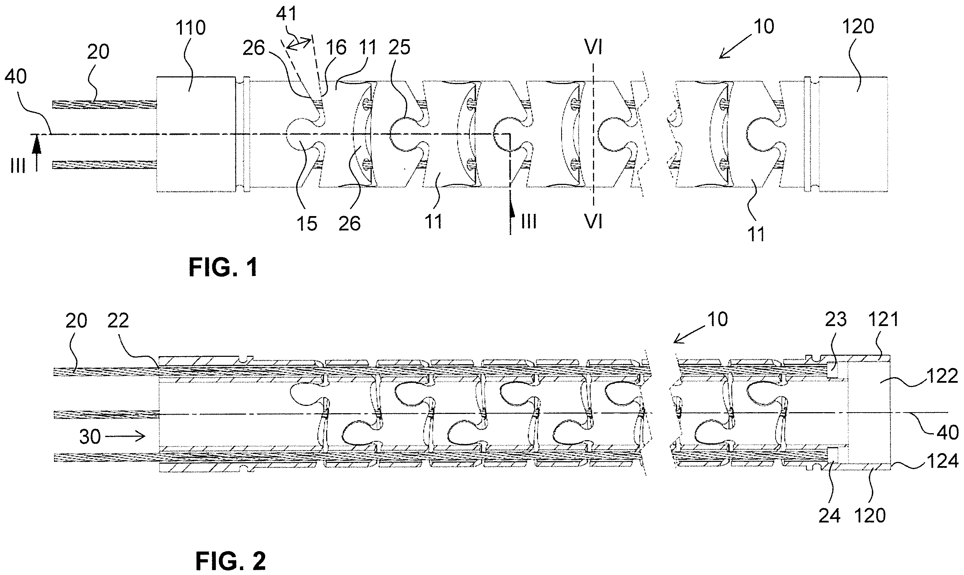

The reception adjacent surface of the cylindrical sleeve of an interconnected link can be inclined in the longitudinal direction of the reception and the protrusion adjacent surface of the cylindrical sleeve of the adjacent interconnected link can be inclined in the longitudinal direction of the protrusion creating the articulating angle between the two surfaces. Then the two surfaces show into the same direction as shown in the embodiment of Fig. 1.

However it is also possible to have a different orientation of the protrusion and reception adjacent surfaces creating larger or smaller gaps of an articulating angle. It is possible that all interconnecting links have the same reception and protrusion adjacent surfaces so that the maximum angle between each pair of adjacent links is the same. On the other side, it is possible to have increasing or decreasing subsequent articulating angles between adjacent links, especially to allow the instrument following a natural lumen of a patient.

A method producing the interconnected links of an articulated segmented instrument produces interconnected links with linear cutting manufacturing processes creating the working channel, the articulation wire thru holes, the protrusions, the receptions as well as the protrusion and reception adjacent inclined surfaces with this process. Such linear cutting manufacturing process are provided by the group comprising laser cutting, electric discharge machining or water jet cutting.

Compared to the prior art this segmented instrument has structurally robust segments at smaller scales, especially smaller than 10mm diameter. The segments being made in one single piece each allow a simplified manufacturing process and avoid cumbersome bridging connections.

A central inner working channel and joined surfaces can be machined from a single stock material.

The segmented instrument according to the invention provides a high torsional and axial stability about the working channel access.

The control of the segmented multi-degree of freedom steerable instrument is controlled by at least three embedded cables. These cables are used for maneuvering the tip of an endoscope, the tip of a catheter or the tip of the searchable tool itself.

The prior art segmented instruments are limited by the possible miniaturized size and robustness. Such tools usually have a tip and it is often required to apply loads onto the target organs. These applied mechanical loads are naturally transferred into the structure of the segmented instrument.

The multi-segmented instrument according to the invention comprises for each segment a single piece of solid metal tube of the required size with orthogonal joint axes cut through the tube using preferably linear cutting manufacturing processes. This allows for endoscopic stylets of an outer diameter of less than 10mm with a tube wall thickness of 1mm or more. Due to the relatively larger wall thickness, these segments are much more mechanically stable than prior art endoscopic stylets.

The single stock material approach allows using linear cutting manufacturing processes as laser cutting, electric discharge machining or water jet cutting, which gives the opportunity to use these robust stock materials.

Each segment incorporates directly the articulating surfaces and the angulation wire through holes for the angulation wires to be cut directly into the bulk of the stock material. This approach alleviates complex and intricate assembly steps as necessary with prior art segmented instruments and provides axially and torsionally stable segments about the channel axis, i.e. the segment array cannot be pulled apart or twisted.

The maximum possible angulation angle is predetermined by the segment length with curvature of the single elements.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic side view of a segmented instrument according to an embodiment of the invention;
- Fig. 2: shows a cross-sectional view of the segmented instrument of Fig. 1 along line II-II of Fig 6;
- Fig. 3: shows a part cross-sectional view of the articulated segmented instrument along line III-III of Fig 1;
- Fig. 4: shows a side view on the articulated segmented instrument with distally increasing pivot angle; the instrument being according to Fig. 1;
- Fig. 5: shows a perspective view of a segment of the articulated segmented instrument according to Fig 1;
- Fig. 6: shows a cross-sectional view on the distal end of the articulated segmented instrument along line VI-VI of Fig 1;
- Fig. 7A: shows a top view of an articulating segment of a segmented instrument according to an embodiment of the invention;
- Fig. 7B: shows a perspective view of the segment of Fig. 7A;
- Fig. 7C: shows a side view along one joint axis of Fig. 7A;
- Fig. 7D: shows a side view along the other joint axis of Fig. 7A;
- Fig. 8A: shows a top view of an articulating segment of a segmented instrument according to an embodiment of the invention;
- Fig. 8B: shows a perspective view of the segment of Fig. 8A;
- Fig. 8C: shows a side view along one joint axis of Fig. 8A;
- Fig. 8D: shows a side view along the other joint axis of Fig. 8A giving a greater pivot angle than Fig. 7D;
- Fig. 9A: shows a top view of an articulating segment of a segmented instrument according to an embodiment of the invention;
- Fig. 9B: shows a perspective view of the segment of Fig. 9A;
- Fig. 9C: shows a side view along one joint axis of Fig. 9A; and
- Fig. 9D: shows a side view along the other joint axis of Fig. 9A giving an asymmetric Pivot angle about the joint protrusion.

### DESCRIPTION OF PREFERRED EMBODIMENTS

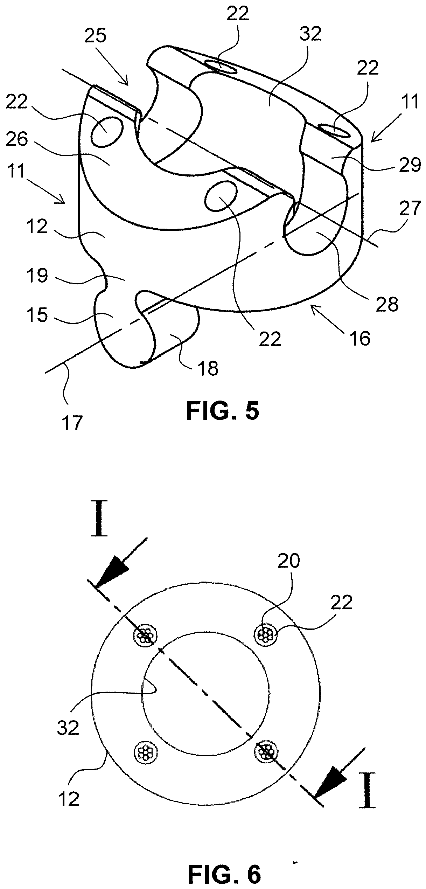

Fig. 1 shows a schematic side view onto a segmented instrument 10 according to an embodiment of the invention. Fig. 2 shows a cross-sectional view along line II-II of Fig 6.

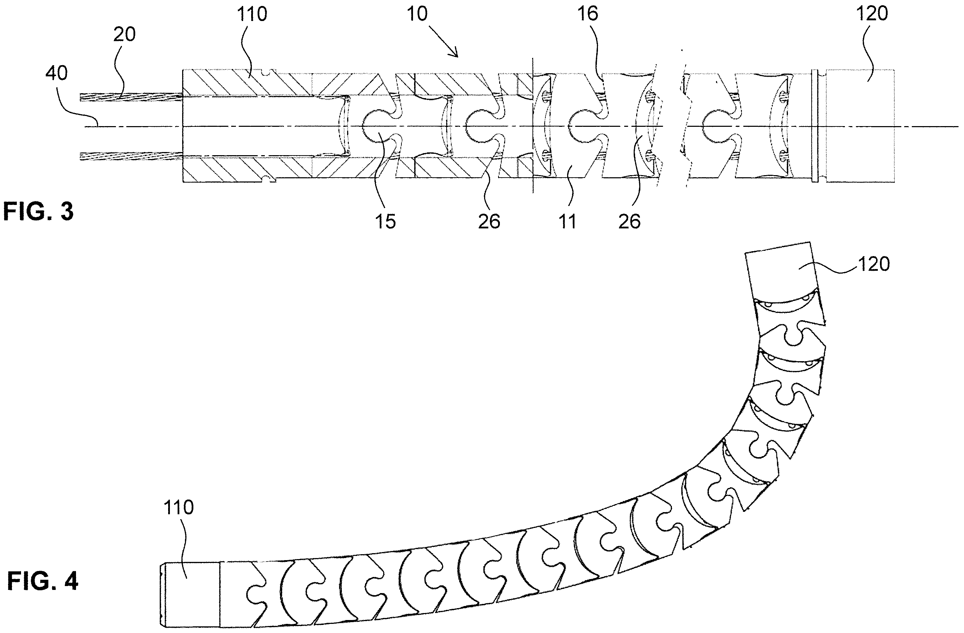

The segmented instrument 10 as shown in Fig. 1 was built as an endoscope with over 40 sequentially arrayed individual articulating segments 11, which have individual varying articulation angles 41 as will be shown in connection with Fig. 4 to achieve an articulation curvature ergonomic to a specific human organ, i.e. with a predetermined sequence of different maximum articulation angles 41.

Four angulation wires 20 are used to steer the instrument 10 about two degrees of freedom. The angulation wires 20 are positioned within the thru going angulation wire thru holes 22 in every segment 11. The angulation wires 20 end in the distal tip of the instrument 10 where a thickened angulation wire end 23 abuts against a shoulder 24 counter boring the thru hole 22 with a larger diameter into the distal tip 120. The angulation wire 20 then passes through all articulating segments 11, followed by passing through the proximal base 110 and continuing beyond the proximal base 110 to an actuator (not shown in the drawings), which is preferably a driven device and not directly manipulated by an operator. The distal tip 120 comprises in a front part a thin outer cylindrical wall 121, which provides a cavity to accept a camera 122. Camera 122 is here closing off the entire inner cavity. Of course, other instruments could be positioned or attached there and also protrude beyond the front surface 124. Instead providing the shoulder and a counter bore, it is also possible to fixate the angulation wires via providing a loop of the wire between two holes 22 instead of using this anchoring method.

Each segment 11 is pivotally connected with a neighboring segment 11 or, at the end portions with the proximal base 110 or the distal tip 120, respectively. The pivotal connection is realized via two protrusions 15 and two receptions 25. These protrusions 15 and receptions 25 will be shown and described in more detail in connection with Fig. 5.

The form of the protrusions 15 is chosen to be on one side complementary to the form of the receptions 25 to allow for a pivotal movement as can be seen in Fig. 4. On the other side they only allow a pivotal movement around one axis. The maximum angle is defined through the opposite surfaces 16 and 26. They create, when the articulating segments 11 are oriented straight along the longitudinal axis 40, an articulation angle 41, which is shown in Fig. 1 between the proximal base 110 and the first articulating segment 11.

Fig. 3 shows a part cross-sectional view of the articulated segmented instrument 10 according to Fig 1. Same numerals indicate identical features. It can be seen that in the sequence of segments 11 every second segment 11 has the same orientation of the protrusions 15. The segment 11 in-between has its protrusions 15 provided in an angle of rotation of 90 degrees around the longitudinal axis 40.

Fig. 4 shows a side view on the segmented instrument 10 according to Fig. 1 bent to a maximum extent in a direction in the drawing plane. Here, the number of segments 11 is twenty-three with an additional distal tip 120 and additional proximal base 110. The angle, as will be shown in connection with Fig. 5, is different for articulated segments 11 near the proximal base 110 compared to the angle between the surfaces 16 and 36 providing the articulating angle 41 between adjacent articulated segments 11 nearer to the distal tip 120.

Fig. 5 shows a perspective view of one single articulated segment 11. Beside the surfaces 16 and 26 and their angle compared to the horizontal axis of Fig. 5 according to the joint axes 17 and 27 all articulated segments 11 are identical. There are two protrusions 15 on the joint axis 17. Each of the protrusions 15 has an outer cylinder surface 18 as outer articulating surface and a front and inner surface, which is flush with the outer surface 12 and inner surface 32, respectively, of the working channel 30. The two axes 17 and 27 are skew lines but intersect in a view from above in an orthogonal angle. In other words, the protrusions 15 and receptions 25 of one single articulated segment 11 have an angle of 90 degrees, one from another in the longitudinal direction 40 of the segmented instrument 10 which is the axis perpendicular to both axes 17 and 27. The protrusion 15 has a neck 19 with a smaller diameter compared to the diameter of the protrusion 15 itself. The neck 19 has a complementary surface 29 at the reception 25. Beyond the neck 19 extends the inner complementary articulating surface 28 in the wall of the segment 11. The surfaces of the protrusion and the recession are simply in contact with one another.

The angulation wire thru holes 22 are ending on the inclined surfaces 16 and 26. In the embodiment of Fig. 1 there are four such holes 22 in each segment 11, which are also provided in an angular distance of 90 degrees, one in respect to each adjacent one. Preferably the holes 22 are provided at the 45 degree intersecting line between the joint axes 17 and 27 for a symmetrical layout. It is noted that only three holes 22 are mandatory, since only three angulation wires 20 are necessary to direct the articulating segmented device 10 into each directions but for reasons of symmetry it is preferred to have these four holes 22 with four wires 20.

Fig. 6 shows a cross-sectional view on the distal end of the articulated segmented instrument along line VI-VI of Fig 1. The wall thickness of each articulating segment 11 is defined as the distance between the outer surface 20 of the segment 11 from the inner surface 32. The outer diameter of the segment 11 can be chosen as small as 5 or 10 millimeter. The inner diameter of such a segment 11 can be chosen to be 3 or 8 millimeter, thus defining the wall thickness to be 1 millimeter. In other embodiments smaller outer diameters can be chosen as e.g. 3 millimeter and the inner diameter can be 1 millimeter with a wall thickness of 1 millimeter as well.

The angulation wire 20 is shown as a wire rope comprising several strands of wire. As it can be seen in this Fig. 6, the angulation wire thru holes 22 have a larger diameter by 30 to 50 % of the average diameter of the angulation wire in order to allow easy bending and pivoting at the surfaces 16 and 26, when the thru holes 22 end.

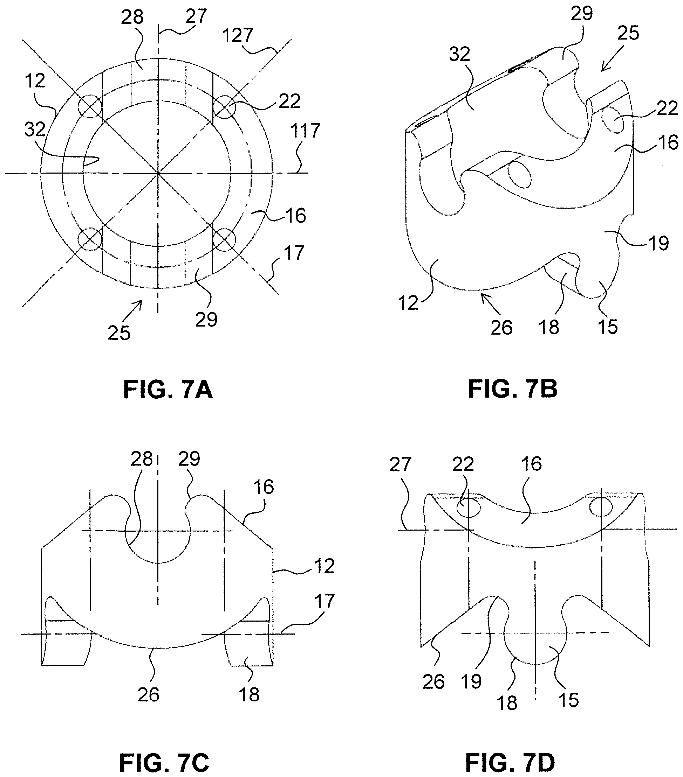

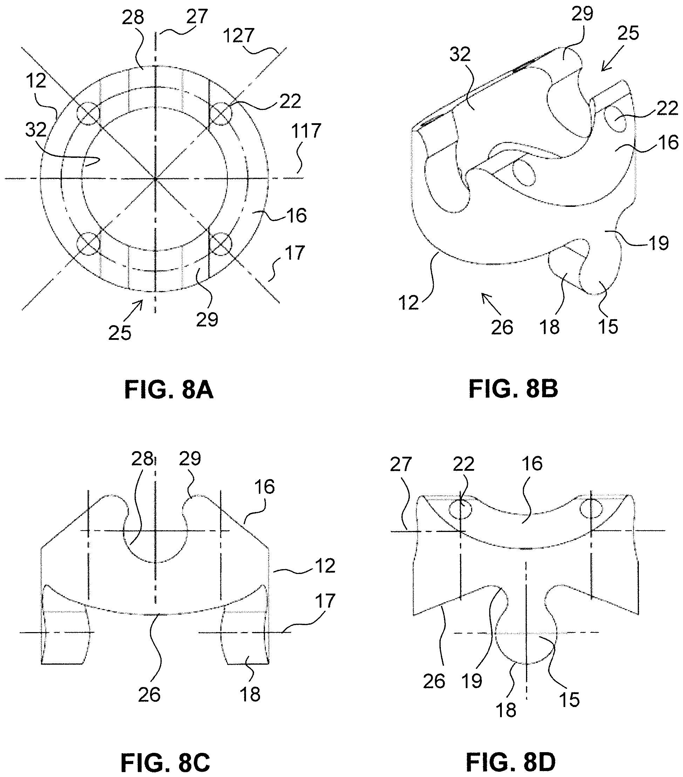

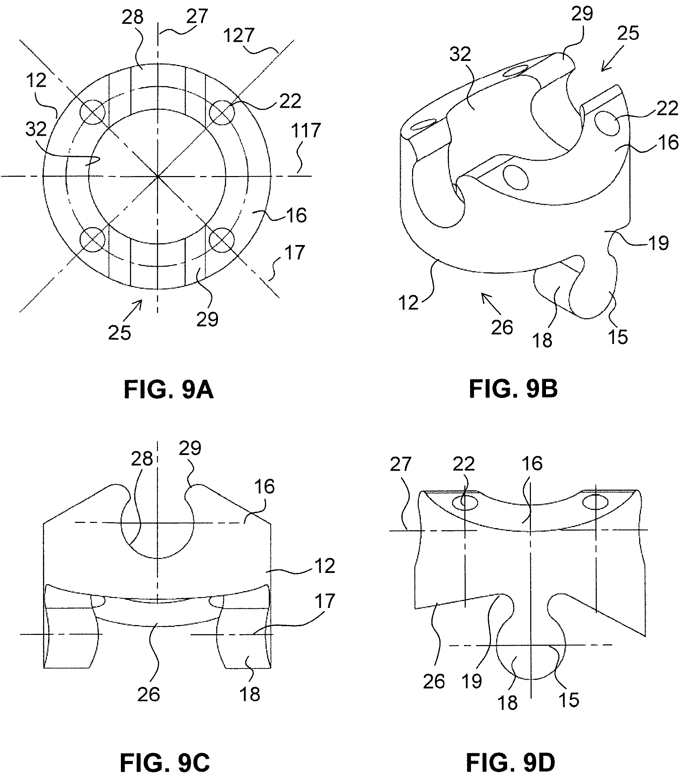

Fig. 7A, Fig. 7B, Fig. 7C and Fig. 7D show each different views of the same articulating segment 11 near the proximal base 110. i.e. a cross-sectional view in Fig. 7A, a perspective view in Fig. 7, a side view similar along to axis 27 in Fig. 7C and a side view similar along to axis 17 in Fig. 7D. The segment 11 shown in the different Figs. 7A to 7D is a segment 11 near the proximal base 110 since it has a steeper angle of the angled surface 16 (being opposite to a complementary angled surface 26 of the adjacent articulated segment 11 or the proximal base 110) compared to the views of Fig. 8A to 8D. Fig. 8A, Fig. 8B, Fig. 8C and Fig. 8D show the same views as Fig. 7A to 7D but for an articulated segment 11 in the distal of the segmented instrument 10. Therefore same or similar angled surfaces 26 are opposing angled surfaces 16 of the adjacent articulating segments 11 with a slope being less steep. Finally, Fig. 9A, Fig. 9B, Fig. 9C and Fig. 9D are similar views on a further segment 11 from the elements near the distal tip 120 having even less steep angled surfaces 16 and 26 and additionally representing an asymmetric segment having different limited bending angles in the different bending directions, i.e. both bending angles on the protrusion side and/or as well as between both maximum bending angles on the reception side. All four angles can be provided having a different maximum pivoting angle with identical complementary surfaces. All reference numerals are provided in the identical way since the elements 11 are identical with the only difference of the angle between the horizontal planes as defined through axes 17 and 27 and the surfaces 16 and 26. The angle between the horizontal plane, which is also defined by the line VI-VI in Fig. 1, and the angled surface 16 is the value of the articulating angle 41 plus the value of the angle between the horizontal plane and the angled surface 26 of the adjacent segment 11.

It can be seen in the side views of Fig. 7C, 8C and 9C that the protrusions 15 have a rounded outer surface following the circumferential surface of the cylindrical jacket 18. The same is true of the cutout of the receptions 25, which can be seen on and around the axis 27 in Fig. 7D, 8D and 9D. Of course, the protrusion 15 complement the cutout for a full cylinder jacket at this place so that no portions are extending outside the diameter defined by the hollow cylinder providing the single body of each articulating segment or link 11.

**LIST OF REFERENCE SIGNS**

| | | | |
|---|---|---|---|
| 10 | segmented instrument | 27 | reception joint axis |
| 11 | articulating segment / link | 28 | reception articulating surface |
| 12 | outer surface | 29 | passage / complementary surface |
| 15 | protrusion | | |
| 16 | angled surface / protrusion adjacent surface | 30 | working channel |
| | | 32 | inner surface |
| 17 | protrusion joint axis | 40 | longitudinal axis |
| 18 | protrusion articulating surface | 41 | articulating angle |
| 19 | neck | 110 | proximal base |
| 20 | angulation wire | 117 | first thru hole position axis |
| 22 | angulation wire thru hole | 120 | distal tip |
| 23 | thickened angulation wire end | 121 | cylindrical wall |
| 24 | counter bore | 122 | camera |
| 25 | reception | 124 | front surface |
| 26 | angled surface / reception adjacent surface | 127 | second thru hole position axis |

## Claims

1. An articulated segmented instrument (10) comprising an elongate body in the longitudinal direction (40) of the segmented instrument (10) between a proximal base segment (110) and a distal tip segment (120) having a plurality of interconnected links (11) between the proximal base segment (110) and the distal tip segment (120), **characterized in that** the interconnected links (11) are hollow cylindrical sleeves with a central working channel (30) and comprising at least three angulation wire thru holes (22) to accommodate each one angulation wire (20) to direct the distal tip segment (120) in a predetermined direction, **in that** each interconnected link (11) comprises two radially opposite protrusions (15) in the cylindrical sleeve extending in the longitudinal direction (40) and having a common joint axis (17) defining a convex pivot contact surface (18) over an angle area of more than 180 degrees around the common joint axis (17) providing a neck portion (19) in the longitudinal direction (40), **in that** each interconnected link (11) comprises two opposite receptions (25) in the cylindrical sleeve extending in the longitudinal direction (40) opposite to the direction of the protrusions (15) and having a common joint axis (27) defining a concave pivot contact surface (28) over an angle area of more than 180 degrees around the common joint axis (27) providing a passage portion (29), wherein the pivot concave contact surface (28) is complementary to the convex pivot contact surface (18) and wherein the neck portion (19) is complementary to the passage portion (29), **in that** the reception adjacent surface (26) of the cylindrical sleeve creates an articulating angle (41) with the protrusion adjacent surface (16) of the cylindrical sleeve of the adjacent interconnected link (11) through inclination of at least one of the reception adjacent surface (26) or the protrusion adjacent surface (16) of the adjacent interconnected link (11) in the longitudinal direction towards the body of the interconnected link (11) or the adjacent interconnected link (11), respectively, and **in that** the protrusion adjacent surface (16) of the cylindrical sleeve creates an articulating angle (41) with the reception adjacent surface (26) of the cylindrical sleeve of the adjacent interconnected link (11) through inclination of at least one of the protrusion adjacent surface (16) or the reception adjacent surface (26) of the adjacent interconnected link (11) in the longitudinal direction towards the body of the interconnected link (11) or the adjacent interconnected link (11), respectively.

2. The articulated segmented instrument (10) according to claim 1, wherein the common joint axis (17) of the protrusions (15) has an angle of 90 degrees with the common joint axis (27) of the receptions (25).

3. The articulated segmented instrument (10) according to claim 1, wherein the common joint axis (17) of the protrusions (15) has an angle of 60 and 120 degrees with the common joint axis (27) of the receptions (25), respectively.

4. The articulated segmented instrument (10) according to any one of claims 1 to 3, comprising four angulation wire thru holes (22) to accommodate each one angulation wire (20) and wherein the angulation wire thru holes (22) are provided in an angle of 90 degree in the longitudinal direction (40).

5. The articulated segmented instrument (10) according to any one of claims 1 to 4, wherein the reception adjacent surface (26) of the cylindrical sleeve of an interconnected link (11) is inclined in the longitudinal direction of the reception (25) and the protrusion adjacent surface (16) of the cylindrical sleeve of the adjacent interconnected link (11) is inclined in the longitudinal direction of the protrusion (15) creating the articulating angle (41) between the two surfaces.

6. A method producing the interconnected links (11) of an articulated segmented instrument according to any one of the preceding claims, wherein each interconnected link (11) is produced with linear cutting manufacturing processes creating the working channel, the articulation wire thru holes, the protrusions, the receptions as well as the protrusion and reception adjacent inclined surfaces.

7. The method according to claim 6, wherein the linear cutting manufacturing process is provided by the group comprising laser cutting, electric discharge machining or water jet cutting.
